# EUROPEAN PATENT APPLICATION

(11) **EP 1 941 857 A2**
(43) Date of publication of application: **09.07.2008**
(21) Application number: 07254953.8
(22) Date of filing: 16.08.2004
(51) Int. Cl.: A61H 21/00

(54) **Apparatus and method for stimulation of the human body**

(30) Priority: 15.08.2003 WO PCT/GB03/19284
(62) Divisional of application: 04768059.0
(71) Applicant: ISIS INNOVATION LIMITED, Summertown, Oxford OX2 7SG (GB)
(72) Inventor: Fairbank, Jeremy, Oxford OX3 7LD (GB); McCulloch, Malcolm, Oxford OX1 3PJ (GB)
(74) Representative: Strachan, Victoria Jane

(57) **Abstract**

Apparatus for stimulation of the human body which comprises an array of vibration actuators 2 which are operated in an array to provide stimulus that the user can sense. There is a user interface 5 including a display 6 having a grid of zones. The interface is connected to a controller 4 which can be used to operate the actuators 2 to train and test a patient.

## Description

The present invention relates to a technique for stimulation of the human body, and particularly to a proprioceptive technique aimed to relieve musculo-skeletal pain.

"Cartesian pain" is the conventional understanding of pain, where pain is assumed to arise from a specific injury or lesion in the body, and experienced in the brain. The Gate Control theory of pain has governed much of recent research in pain since it was published by Melzack and Wall in 1965. Gate Control theory leads directly to the development of transcutaneous nerve stimulation (TENS) as well as too much of our understanding of acute pain and the transition of acute to chronic pain.

Chronic musculo-skeletal pain is poorly understood. The Cartesian model of pain where a specific lesion or injury causes pain does not work well in most patients with chronic pain. This is true for low back pain, cervical pain, particularly whiplash injury, and other forms of spinal pain. It is also the case for chronic shoulder pain, hip pain, knee and ankle and some forms of repetitive strain injury in the upper limb.

In low back pain there is strong support for the idea that muscle function is disorganised and reflex responses to perturbation is abnormal *(*Fairbank, JCT, O'Brien, JP, Davis, PR. Intraabdominal pressure rise during lifting as an objective measure of chronic low back pain. Spine 1980; 5: 179-84*,* Radebold, A, Cholewicki, J, Polzhofer, GK, Greene, HS. Impaired postural control of the lumbar spine is associated with delayed muscle response times in patients with chronic idiopathic low back pain. Spine 2001; 26: 724-730*).* Similar findings have been made for other forms of chronic musculo-skeletal pain.

In the low back there is evidence that physical fitness *(*Frost, H, Klaber Moffett, JA, Moser, JS, Fairbank, JCT. Randomised controlled trial for evaluation of fitness programme for patients with chronic low back pain. Brit Med J 1995; 310:151-154*)* and specific multifidus muscle exercises are effective in relieving pain.

This invention is based on a hypothesis of "cortical pain" formulated by Harris *(*Harris A.J. Hypothesis: Cortical origin of pathological pain. Lancet 1999; 354: 1464-1466*),* which in turn was based on works by Ramichandran *(*Ramichandran, VS, Hirstein, W. The perception of phantom limbs. Brain 1998; 121; 1603-1630*)* and Fink *(*Fink GR, Marshall JC, Halligan PW, Frith CD, Driver J, Frackowiak RSJ, Dolan RJ. The neural consequences of conflict between intention and the senses. Brain 1999; 122: 497-512*)* which suggest that the cerebral cortex has at least once centre which is activated when discordant proprioceptive and visual information are received.

There is evidence that phantom limb pain, even when present for many years, can be eased by the use of proprioceptive training which in turn can lead to cerebral cortical reorganisation *(*Flor H, Denke C, Schaefer M, Grusser S. Effect of sensory discrimination training on cortical reorganisation and phantom limb pain. Lancet 2001; 357: 1763-1764*).*

According to the present invention, there is provided apparatus for stimulation of the human body, the apparatus comprising:
an array of stimulator elements arranged to be operated in a plurality of stimulator activation zone configurations; and
a user interface device permitting the user to relate interface zones of the interface device to activation zones of the array of stimulator elements.

According to an alternative aspect, the present invention provides a method of stimulation of the human body, the method comprising stimulating the surface of the body with an array of stimulator elements, the array being operated to activate an activation zone configuration from a plurality of potential activation zone configurations, wherein the user interfaces with an interface device such that interface zones of the interface device correlate to the active actuation zone configuration of the array of stimulator elements.

It is preferred that user input into a spatial input zone array of the interface device effects a corresponding spatial and/or temporal activation of the stimulator element array.

Additionally or alternatively, the activation zone of the stimulator element array is beneficially selected independently of user knowledge, the users input to a spatial input zone array of the interface device being used to identify the user's perception of the activation configuration of the array of stimulator elements.

It is believed that the invention promotes cerebral cortical reorganisation leading to relief of chronic musculo-skeletal pain. This invention is specifically not intended to deliver either painful stimuli or massage. The stimulus is to be localised as possible. Cortical retraining is likely to take several weeks. In contrast, the rationale of other techniques such as transcutaneous nerve stimulation (TENS) is to deliver afferent input which is supposed to close the "gate" in the cells of the dorsal horn, thus blocking the central sensation of pain. Traditional treatments such as massage, hot and cold stimuli may work in a similar way *(*Wall, P. Pain: the science of suffering. London: Weidenfeld and Nicholson, 1999*)*.

By contrast, the present invention relies on teaching proprioceptive techniques to patients with a view to altering cerebral cortical re-organisation in patients with chronic musculo-skeletal pain. The present invention educates subjects in the localisation of stimuli on the skin innervated by the same nerves as innervate the deep structures of the painful area. It does not attempt to give position or movement data of joints.

Typically, an activation zone of the array corresponds to the zone physically influenced by a single stimulator element. This does not however preclude the possibility that an activation zone may include a zone physically influenced by more than one stimulator element. Likewise, the actuation configuration for the sensor array will normally involve only one stimulator element being active at any one time.

It is preferred that the apparatus is configured to permit spatial and/or temporal correlation between the zones of the interface device and the active zones of the activation zones of the array of stimulator elements.

Beneficially, the interface device includes a spatial input and/or output zone array corresponding to the positional spacing of activation zones of the array of stimulator elements.

It is preferred that the interface device includes a screen providing output data and/or permitting user input relating to the activation zones of the array of stimulator elements. It is preferred than the apparatus is operable in a mode in which user input into the interface device determines the actuation zone configuration of the array of stimulator elements.

Additionally or alternatively, the apparatus is preferably operable in a mode in which the activation zone configuration of the array of stimulator elements is selected without influence of the user and the user uses the interface device to identify the activation configuration as perceived by the user.

Beneficially the apparatus permits switching between modes.

It is preferred that the apparatus includes means for storing results data. Results data may be transmitted to a remote store or processor.

Beneficially, in one embodiment the array of stimulator elements are carried in a predetermined spatial relationship on a support member. The support member may comprise a garment to be worn by the user. For example, where the apparatus is used by a person with back pain, the support member for the array may be a corset to be worn by the user positioning the array of stimulator elements against the relevant portion of the back of the user.

In an alternative embodiment, the array of stimulator elements are implanted in a spatial relationship in the body of an individual that is to be monitored by the device. The stimulator elements may be implanted individually or they may be mounted on a carrier which itself is implanted in the body of the individual.

Beneficially, barrier zones are provided about the stimulator elements to inhibit transmission of the physical stimulus (through the support) beyond the locality of the relevant stimulator element. The stimulator elements may be vibrator actuators for example and barrier boundaries may be provided about the stimulator elements to maximise attenuation of the physical stimulus.

It is preferred that the stimulator elements are arranged grid-wise in rows and columns. Other configurations may however be utilised. Beneficially, input zones of the interface device are arranged in corresponding grid-wise rows and columns. The input zone of the interface device may be a touch screen or the like divided into cells or zones.

The sensor array is beneficially operated such that, generally only one stimulator element is activated at one time. The length of time of operation of the stimulator element may be selectively varied. The stimulation intensity of the stimulator elements may, preferably, be selectively varied.

It is preferred that the apparatus includes communication means to receive signals and/or transmit data to data processing means such as a computer.

Preferably, the signals are radiowaves or microwaves. It is envisaged that the apparatus can receive signals from a palmtop or a lap top computer or even from a mobile phone with WAP technology.

It is preferred that the apparatus can process signals from the individual and transmit these to a remote processor such as that contained in for example a palmtop, a lap top, a mobile phone, or a custom built device that can process data received.

It is preferred that the data collected can be periodically or continuously monitored in order to study the pain experienced by an individual over a defined time period. Data can be stored to study whether the pain is experienced at certain times of the day or results from the individual being in different situations and these data can be used as a means of establishing whether the pain is triggered under certain conditions.

The invention will now be further described in a specific embodiment, by way of example only and with reference to the accompanying drawings in which:
Figure 1 is a schematic representation of apparatus for use in accordance with the invention; and
Figure 2 is a schematic representation of an interface device having four interface zones selected.

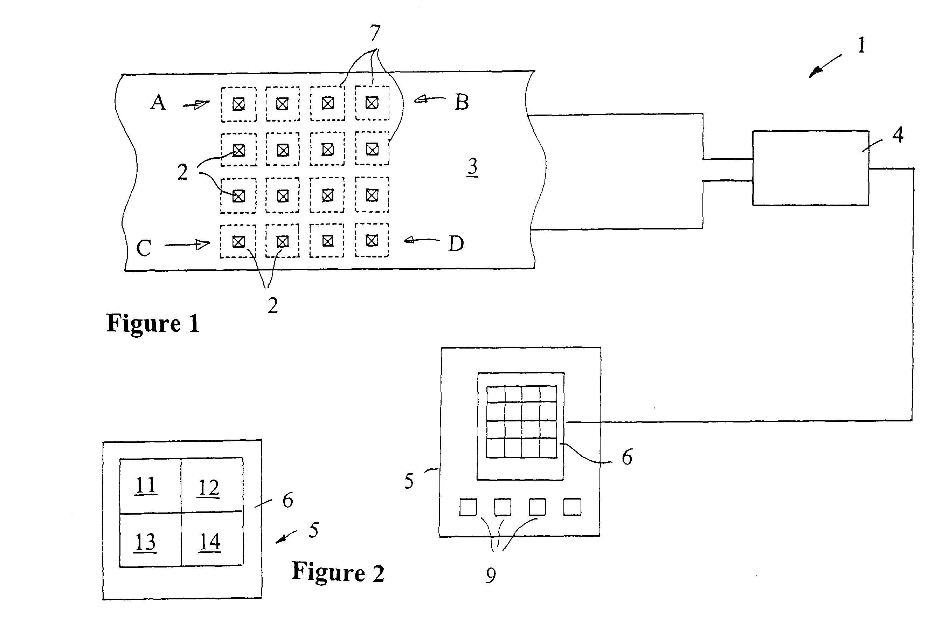

Referring to the drawings, there is shown apparatus 1 comprising an array of vibration actuators 2 mounted to a substrate corset 3. The actuators are arranged in a 4x4 grid of rows and columns with a separation of 50mm between centers. A user interface control device in the form of a hand held computer device 5 includes a touch screen display 6. Interface device 5 is connected to an operation controller 4 to supply power to activate the vibration actuators 2 of the corset 3. The touch screen 6 of the hand held computer 5 in Figure 1 has a grid of 16 zones correlating to the 16 vibration actuators 2 mounted in the corset 3.

The function of the vibration actuator 2 array is to provide a stimulus that the user can sense. In particular the stimulus should be a localized sensation.

Each vibration actuator 2 comprises a small dc motor which has an integral eccentric rotor, providing the stimulus. When operated the actuator therefore vibrates. This type of motor is typically used in mobile phones and pagers. They require about 3V to operate. By changing the voltage the level of vibration changes.

The motors are housed in a semi-rigid material of the corset 3. They are placed with the motor axis parallel to the material. This maximizes the vibration to the user. In order to ensure that the stimulus is local, a boundary 7 is cut away around each actuator 2 in the form of a rectangle. This minimizes the mechanical energy transferred to the rest of the supporting material of the corset 3.

Each actuator 2 motor requires two wires, positive and negative. To simplify the wiring harness, all the positive terminals are connected together, and only one wire is required to connect to the positive terminal of the operation controller 4. The negative terminal of each motor is connected independently to the operation controller 4, allowing for the operation of each motor independently of the others. The 16 motors are connected to two cables of 8 wires, the earth shielding carrying the positive return. The wires are terminated in 9-way male D-connectors that plug into the operation controller 4.

The function of the operation controller 4 is to convert the signal from the hand held computer device 5 into a voltage which can power a particular vibration actuator 2 motor.

A micro controller controls the operation of the operation controller 4. A request to turn on or off a particular vibration actuator 2 motor is generated by the hand held computer device 5 and is communicated to the operation controller 4. The micro-controller interprets the command, and then turns on (or off) an appropriate transistor in a transistor array in the operation controller 4. The transistors are configured as simple power switches, which then apply power from the battery to the appropriate motor. The voltage regulator converts the 9V of the battery to 5V required by the micro-controller.

When a signal comes from the hand held computer 5 serial communications interface, a driver converts the voltage to levels suitable for the micro-controller.

The actuators 2 can be operated in high or lower intensity mode. A higher intensity mode should be used initially by the user. Lower intensity vibration can be used as the user's sensitivity to the stimulus improves.

The hand held computer 5 is used to control the operation controller 4 and therefore the operation of the vibration actuators 2 is the grid array of the corset 3. The system can be used in two modes to train and test the patient.

The test mode randomly (independently of user input) selects one of the vibration actuators 2, activates it and then waits for the patient to identify the correct position. The train mode allows the patient to select which vibration actuator to activate. in both these modes 2, 4, 8 or 16 actuators can be selected. In order to simplify explanation, Figure 2 shows the touch screen 6 of the interface hand held computer 5 displaying four touch zones 11, 12, 13, 14 corresponding to four actuators 2 only (for example A, B, C, D - in Figure 1) which are active for the corset 3.

The training mode of the apparatus permits the user to activate the vibration actuators 2 so as to learn how to distinguish different sensory areas of the body (in this specific example, different sensory areas of the back). Figure 2 shows a four touch zone training screen of the device used in an example in which four active vibration stimulator 2 zones have been selected to be potentially active. Three extra input buttons 9 are utilised in addition to the four touch zones 11, 12, 13, 14 of the screen. The four touch zones of the screen correspond spatially to the four active actuator zones of the vibration actuator array 2. For example, as in the present instance where four zones are selected, the four touch zones 11, 12, 13, 14 of the screen can correspond spatially to four correspondingly spatially arranged actuatable vibration actuators 2 in the array (for example actuators A, B, C, D) in Figure 1. The widely spaced group of four actuators A, B, C, D could be used in initial training or testing; a more closely spaced group of sensors 2 could be used as the user's performance improves. The apparatus permits from a minimum of two to a maximum of sixteen actuatable vibration actuator zones and corresponding touch input zones on the computer interface device screen 6.

Each touch zone on the screen 6 corresponds to a vibration actuator 2 on the corset 3. Tapping the screen touch zone selects the corresponding actuator 2 causing it to vibrate for a fixed length of time. The duration of the vibration event and the intensity can be increased or decreased dependent upon user requirements. Input to the hand held device 5 can be used to achieve this. Typically the minimum vibration duration selectable is 0.25s and the maximum 12s. The memory of the hand held computer device 5 records the results in train mode. The user is able to relate the spatial position of the screen touch zone to the physical stimulus applied by the active vibration actuator.

In the test mode of the apparatus, a random (not user selected) vibration actuator 2 in the array on corset 3 is caused to vibrate for a predetermined duration at a predetermined intensity. Tapping the relevant touch zone 11, 12, 13 14 on the screen 6 of the hand held computer identifies which actuator the patient believes was actuated. the patient perceived actuator is compared to the actual actuator and an indication of success result is given to the patient (either visually or audibly or by both means). A running total of results is stored permitting subsequent or real-time analysis. The results from test or train modes can be downloaded to a PC or other processor for analysis of results. Alternatively, data may be downloaded in real-time by means of wire or wireless connections to a PC or other processor, for example a palmtop, a lap top or a mobile phone.

As previously mentioned, the apparatus may comprise an array of stimulator elements that are implanted in the body. The stimulator elements would be encapsulated in a bio-compatible material, using for example a biocompatible cable, foil or plastic. In order to receive or transmit data the apparatus includes a read/write probe (not shown) that can read information/signals received and compile this data which can be transmitted to receiving means such as a computer. The data can be read using a portable computer such as a lap top or palm top which has benefits in that individuals can be seen either at home or at different locations such as a clinic or at a research facility. The data can then be stored and sent to a central computer for example held at a hospital which can receive data from a number or external units so allowing for studies of individuals from different localities.

It will readily be appreciated by the skilled addressee that variations may be made from the described embodiments without departing from the scope of the invention. In particular, the interface device may have minimal interaction with the user, in a most simple embodiment simply displaying visually positioned data corresponding to the position of an active stimulator element (vibration actuator).

## Claims

1. Apparatus for stimulation of the human body, the apparatus comprising:
an array of stimulator elements arranged to be operated in any selected one of a plurality of stimulator activation zone configurations so as to provide repeated localised external stimulation at one or more locations on the human body corresponding to the selected zone configurations so as to provide repeated localised external stimulation at one or more locations on the human body corresponding to the selected activation zone configuration; and
a user interface device permitting the user to relate interface zones of the interface device to activation zones of the array of stimulator elements.

2. Apparatus according to claim 1, wherein the interface device permits spatial and/or temporal correlation between the zones of the interface device and the active zones of the activation zones of the array of stimulator elements; and/or wherein the interface device includes an interface zone array corresponding to the positional spacing of activation zones of the array of stimulator elements.

3. Apparatus according to any preceding claim, wherein the interface device includes a screen providing output and/or permitting user input relating to the activation zones of the array of stimulator elements; and/or wherein the apparatus is operable in a mode in which user input to the interface device determines the activation zone configuration of the array of stimulator elements; and optionally wherein the interface device includes an input zone array corresponding spatially to the activation zone configuration of the array of stimulator elements.

4. Apparatus according to any preceding claim, wherein the apparatus is operable in a mode in which the activation zone configuration of the array of stimulator elements is selected independently of the user and the user uses the interface device to identify the activation configuration as perceived by the user; and optionally wherein the interface device includes a selectable array of input zones corresponding to the activation zone array of the stimulator elements.

5. Apparatus according to claim 4, permitting switching between modes.

6. Apparatus according to any preceding claim further including means for storing results data.

7. Apparatus according to any preceding claim, wherein the array of stimulator elements are carried in a predetermined spatial relationship on a support member, and optionally wherein the support member comprises a garment to be worn by the user; and optionally wherein the garment comprises a corset to be worn by the user; or wherein the support member comprises an implant for insertion in the body.

8. Apparatus according to claim 7, wherein the support member includes barrier zones about the stimulator elements to maximise attenuation beyond the locality of the stimulator elements.

9. Apparatus according to any preceding claim, wherein the stimulator elements are arranged grid-wise in rows and columns.

10. Apparatus according to any preceding claim, wherein the stimulator elements comprise vibrator devices; and/or wherein the stimulation intensity of the stimulator elements can be varied; and/or wherein the activation duration of the stimulator elements can be varied.

11. Apparatus according to any preceding claim including a control arrangement to control the interaction between the interface device and the stimulator element array; and/or wherein the apparatus includes data transmitting means whereby results from the apparatus can be downloaded to a processor by wire or wireless connections; and optionally wherein the processor forms part of on or more of: a personal computer, a palm top computer, a lap top computer, a mobile phone, or a custom built device.
